# EUROPEAN PATENT SPECIFICATION

(11) **EP 1 950 213 B1**
(45) Date of publication and mention of the grant of the patent: **05.08.2009**
(21) Application number: 08008434.6
(22) Date of filing: 31.01.2005
(51) Int. Cl.: C07D 487/04, C07C 233/43

(54) **N-{3-[3-(dimethylamino)-1-oxo-2-propenyl]phenyl}-N-ethyl-acetamide hydrochloride**
N-{3- [3-(dimethylamino)-1-oxo-2-propenyl]phenyl}-N-ethyl-acetamide hydrochlorid
Hydrochloride de N-{3- [3-(diméthylamino)-1-oxo-2-propényl]phényl}-N-éthyl-acétamide

(30) Priority: 02.02.2004 HU 0400325
(43) Date of publication of application: 30.07.2008
(62) Divisional of application: 05708499.8
(73) Proprietor: Richter Gedeon Nyrt., 1103 Budapest (HU)
(72) Inventor: Czibula, László, 1103 Budapest (HU); Dobay, László, 1073 Budapest (HU); Szöke, Katalin, 1105 Budapest (HU); Wernké Papp, Éva, 1103 Budapest (HU); Nagynè Badgy, Judit, 1119 Budapest (HU); Tàrkànyi, Gábor, 2040 Budaörs (HU)
(74) Representative: HOFFMANN EITLE

(56) References cited:
- WO-A-02/100828

## Description

The compound of formula (IV) (zaleplon) is an anxiolytic, antiepileptic, sedative, hypnotic, and skeletal muscle relaxing agent acting as a GABA_{A} receptor agonist.

The compound of formula (IV) is described in the US patent No. 4,626,538 in the Example 14 in Table 8. The final product is obtained by refluxing the N-[3-[3-(dimethylamino)-oxo-2-propenyl]phenyl]-N-ethyl-acetamide base of formula (I) with the 3-amino-4-pyrazol-carbonitrile base of formula (III) in glacial, acetic acid for 8 h. The exact physico-chemical properties, the purity and the yield of the compound, are not described in this patent, only the melting point is given, which is 186-187 °C.

The process of the US patent No. 4,626,538 is improved in the EP patent No. 776,898. The improved process for the synthesis of the compound of formula (TV) is the following: the N-[3-[3-(dimetilamino)-oxo-2-propenyl]phenyl]-N-ethyl-acetamide base of formula (I) is reacted with the 3-amino-4-pyrazol-carbonitrile base of formula (III) in a mixture of acetic acid and water (from 10 v/v % to 85 v/v %); preferably in a 1:2 v/v mixture of them. The purity of the so obtained product is 98.86-99.40 % according to HPLC. The yield of the purest (99.4 %) compound is 83.5 % as described in Example 1. The starting materials are basis in all of the examples. The process for the synthesis of the compound of formula (IV) is claimed starting not only from the intermediate basis but also from the salts thereof. There is no process described in the above EP patent, which uses a salt as starting material and there are no descriptions of the applicable intermediate salts or the synthesis thereof.

The disclosed patent application No. WO 2002/100828 also describes a process for the synthesis of the compound of formula (IV) comprising the reaction of the N- [3- [3-(dimethylamino)-oxo-2-propenyl]phenyl]-N-ethyl-acetamide base of formula (I) with the 3-amino-4-pyrazol-carbonitrile base of formula (III) in a mixture of water and a water miscible organic solvent under acidic conditions. Acidic conditions are achieved by the addition of organic or inorganic acid - among others hydrochloric acid - to the reaction mixture. The amount of the acid can be equimolar with one of the starting base or can be applied in large excess. In claims 12-15 the application of the acid addition salt of the base of the formula (I) as starting material and the addition of an acid are claimed separately, but on the one hand in the cited claims 6 and 1 only the base is used as starting material, on the other hand the description does not support the application of a salt as a starting material. The patent application No. WO 2002/100828 - similarly to the EP patent No. 776,898 - does not give example or description of the preparation, isolation and preferred embodiment of the salts of bases of formula (I) or (III). In Example 12 one equivalent of hydrochloric acid is used, which is the lower limit of the amount of the applied acid, as claimed in claim 21. According to this example the product is obtained after 24 h reaction time, in 82 % yield and the purity is only 98.95 %. In our experiments - carrying out the synthesis according to the above Example 12 and using one equivalent of hydrochloric acid - the measured pH was around 1.5 during the whole reaction time. In those reactions, where the amount of the applied acid is more than one equivalent - as described in Examples 1-21 - the measured pH was below 1.0; i.e. strongly acidic reaction conditions are used.

In the patent application No. WO 2003/011228 a new regioisomer of the compound of formula (IV) - N-[3-(3-cyanopyrazolo[1,5-a]pyrimidin-5-yl)-phenyl]-N-ethyl-acetamide - is described, which is formed during the synthesis of the compound of the formula (IV). Considerable amount of this regioisomer - 0.2-0.5 % - is formed for example during the process for the synthesis of compound of formula (IV) described in the US patent No. 2003/0040522.

Since the above chemical purity values are lower than those required in the pharmaceutical industry nowadays, our aim was to find a new process for the synthesis of compound of formula (IV), which is suitable for the preparation of high purity (fulfilling the strictest quality requirements) product, in good yield.

The basis of our invention is the following discovery: the pH of the reaction mixture should be kept between 3.0 and 3.5 during the reaction in order to reach the highest selectivity of the chemical reaction. As a result of our research the optimal pH of the mixture, which is required to obtain the high purity compound of formula (IV), is maintained in a self-adjusting way by application of the high purity hydrochloric acid salt of formula (II).

During our research it was also found, that the high purity of intermediates - especially that of the last ones - is a precondition of the high purity of the final product. The high quality 3-amino-4-pyrazol-carbonitril base of formula (III) is commercially available. The base of formula (I) can be obtained in a multistep synthesis, the basic form of the compound of the appropriate purity can only be isolated after loosing great amount of the material. Surprisingly it was found that from among the salts of the base of formula (I) the hydrochloride salt - which has not been characterized before - can easily be obtained from the base of formula (I) in high purity and very good yield.

In the examples of the prior art patents and patent applications dealing with the synthesis of compound of formula (IV) the reaction is carried out under highly acidic conditions, in some cases using more than one equivalent of acid. First we repeated these known procedures. It was found, that under these highly acidic conditions in every case the obtained compound of formula (IV) contained more than 0.2 % of the regioisomer N-[3-(3-cyanopyrazolo[1,5-a]pyrimidin-5-yl)-phenyl]-N-ethyl-acetamide. Then we elaborated a new process for the synthesis of compound of formula (IV) of high purity.

Surprisingly it was found, that the compound of formula (IV) can be synthesized by reacting the new hydrochloride salt of formula (II) of the high purity base of formula (I), which was obtained in good yield by our process, and the base of formula (III) without adding acid to the reaction mixture, moreover the yield and the purity of the product is much higher, than that described in the prior art.

The synthesis of the high purity compound of formula (IV), which contains less than 0.2% impurities, has not been described in the literature, neither a process according to our invention for the synthesis of compound of formula (IV) without adding acid to the reaction mixture.

The invention relates to the new N-{3-[3-(dimethylamino)-1-oxo-2-propenyl]phenyl}-N-ethyl-acetamide hydrochloride of formula (II).

Furthermore the invention relates to a process for the synthesis of the new N-{3-[3-(dimethylamino)-1-oxo-2-propenyl]phenyl}-N-ethyl-acetamide hydrochloride of formula (II) having a purity of more than 99.5 %, comprising of suspending the compound of formula (I) in acetone at 20-25°C under nitrogen and adding equimolar amount of concentrated hydrochloric acid.

The detailed description of the invention is as follows:

The meaning of "at room temperature" is at 20-25 °C.

The meaning of "crude final product" is the final product obtained by reproduction of the procedures for the synthesis of compound of formula (IV) of the prior art.

The meaning of "highly acidic conditions" is carrying out the reaction below pH 1.0.

According to our invention the new salt of formula (II) is obtained by suspending the base of formula (I) in acetone at room temperature under nitrogen and adding equimolar amount of concentrated hydrochloric acid. After stirring the precipitated crystals are filtered off, washed and dried. The purity of the so obtained product is more than 99.5.%.

The synthesis of therapeutically applicable compound of formula (IV) containing less than 0.2 % impurities is the following:

The base of formula (III) is dissolved in a mixture of water and an alcohol and the solid salt of formula (II) is added in small portions to the warmed solution. Vigorous stirring is continued during the addition. According to an other embodiment the salt of formula (II) is dissolved in a solvent and the solution is added to the reaction mixture. First the solution, later on the obtained suspension is stirred continuously, then the product is isolated. Usually the suspension is cooled and stirring is continued. The precipitated crystals are filtered off, washed and dried. The so obtained crystalline material is dissolved in warm methanol under nitrogen, the solution is filtered, cooled, the precipitated product is filtered off, washed and dried.

The process is preferably carried out in an organic solvent or in a mixture of water and a water miscible organic solvent. As organic solvent short chain aliphatic alcohols, preferably methanol, ethanol, isopropanol, n-propanol can be used. The process according to our invention is preferably carried out at 15-50 °C, for 0.5-1 h. The 3-amino-4-cyano-pyrazole base of formula (III) is preferably dissolved in an appropriate mixture of solvents and the hydrochloride salt of formula (II) is added to the solution in small portions. After cooling the precipitated product is filtered off and dried. The purity of the so obtained product of formula (IV) is more than 99.8 % according to HPLC.

Application of the process of our invention - reacting the new, high purity hydrochloride salt of formula (II) and the compound of formula (III) - results in a much higher quality (purity more than 99.4 %) compound of formula (IV), than the one can be obtained according to the prior art, i.e. carrying out the reaction in aqueous acetic acid.

The high purity, which is especially advantageous in the pharmaceutical industry, of the final product of formula (IV) is partly due to the high purity of the new hydrochloride salt of formula (II), partly to the acid free reaction mixture, partly to the addition of the new hydrochloride salt of formula (II) in small portions and partly to the short reaction time.

The great advantage of the application of the new hydrochloride salt of formula (II), which was first prepared and used by us in the synthesis of compound of formula (IV), is that a high purity starting material is used in the synthesis of compound of formula (IV) of high purity - required in the pharmaceutical industry. According to HPLC, the purity of the base of formula (I) is generally 90-95 %, while the purity of the hydrochloride salt of formula (II) obtained from this base is more than 98.5 %.

It has to be emphasized that during the process for the synthesis of compound of formula (IV) the pH of the reaction is 3.0-3.5. The great advantage of our process is that it is not necessary to add further amount of acid to the reaction, because the new hydrochloride salt of formula (II) used as starting material contains equimolar acid, moreover the pH of the reaction mixture remains between 3.0-3.5 during the whole reaction time, due to the addition of the hydrochloride salt of formula (II) in small portions to the reaction mixture, consequently the reaction conditions of our process for the synthesis of compound of formula (IV) are much milder compared to the procedures of the prior art, in which the reaction is carried out below pH 1.0 or at most at pH 1.5.

The great advantage of the application of the new hydrochloride salt of formula (II), which was first prepared and used by us in the synthesis of compound of formula (IV), is that the regioisomer of compound of formula (IV) is formed only in less than 0.1 % yield. The final product of formula (IV) prepared according to the procedures of the prior art contains - as declared in the descriptions - in some cases 0.5 % ofregioisomer as impurity.

Further advantage of the application of the new hydrochloride salt of formula (II), which was first prepared and used by us in the synthesis of compound of formula (IV), is besides the formed small amount of the regioisomer of formula (IV), that the impurity of formula (V), which has not been mentioned in the literature or in the disclosed patents and was isolated by us, is formed only in traces (0.07 %).

The analytical characterization of the impurity of formula (V) is given in Example 2. Our analytical measurements proved the structure of the compound of formula (V), its chemical name is N-{3-[3-cyanopyrazolo(1,5-a)pyrimidin]-6-yl-3-[(3-N-ethyl-acetamidophenyl)-3-oxo-propene-1-yl]-(pyrimidin-7-yl)-phenyl}-N-ethyl-acetamide.

The above mentioned impurity of formula (V) could be isolated from the crude final products obtained according to the methods described in the literature for the synthesis of compound of formula (IV) as these crude products contained larger amount (0.1 %) of the impurity of formula (V). The impurity of formula (V) was isolated from the mother liquor of the recrystallization by preparative HPLC. Surprisingly it was found that the impurity of formula (V) is formed only in 0.07 % yield in the process of our invention for the synthesis of compound of formula (IV).

We realized that the formation of the impurity of formula (V) can be diminished by addition of the hydrochloride salt of formula (II) in small portions to the reaction mixture during the process of our invention for the synthesis of compound of formula (IV), as well as by keeping the appropriate pH value, temperature, ratio of solvent and reaction time. This is especially advantageous in the pharmaceutical industry.

In summary the advantages of the process of our invention for the synthesis of compound of formula (IV) in comparison to the known ones are as follows:
- the final product of formula (IV) is prepared in good yield, containing less than 0.2 % impurities according to GMP requirements of pharmaceutical drug manufacturing;
- the purity of the hydrochloride salt of formula (II) is very high;
- the final product of formula (IV) is prepared without addition of acid;
- the final product of formula (IV) contains much less (max. 0.1 %) regioisomer as impurity compared to the ones obtained according to procedures of the prior art;
- the final product of formula (IV) contains much less (0.07 %) impurity of formula (V), which was isolated by us, compared to the ones obtained according to procedures of the prior art;
- the high purity of the drug can safely be controlled during manufacturing of the final product of formula (IV) as not only the high purity of the final product of formula (IV) but that of the final intermediate of formula (II) can also be validated.

The following examples are illustrative and are not meant to limit the scope of the claimed invention.

In the examples the new compound of formula (II), the final product of formula (IV) and its impurity of formula (V) obtained according to the process of our invention are characterized by the following analytical methods.

The purity of the product obtained according to the process of our invention was determined by HPLC. The HPLC apparatus: Thermo Separation Products, pump: Spectra System P2000, detector: Spectra System UV2000. Control, data collection and data processing: ChromQuest program, Ver. 2.51. Conditions of chromatography: column: YMC-Pack Pro C18, 150x4.6 mm ID, 5 µm. Eluent A: a mixture of 140 ml of acetonitrile + 30 ml of methanol + 30 ml of tetrahydrofuran + 800 ml of 0.1 M ammoniumacetate, eluent B: a mixture of 400 ml of acetonitrile + 100 ml of methanol + 100 ml of tetrahydrofuran + 400 ml of 0.1 M ammoniumacetate, from these a linear gradient solvent system was used, which is given in Table 1.

Flow rate: 1 ml/min; temperature of the column: room temperature; wavelength of detection: 235 nm. Dissolution of the analyzed sample: in a mixture of 77 % of eluent A + 23 % of eluent B. Injected amount: 10 µl.

**Table 1**

| HPLC gradient solvent system | | |
|---|---|---|
| Time (min) | Eluent (v/v %) | |
| | A | B |
| 0 | 77 | 23 |
| 15 | 77 | 23 |
| 20 | 50 | 50 |
| 25 | 0 | 100 |
| 25.5 | 77 | 23 |
| 31 | 77 | 23 |

¹H-NMR and ¹³C-NMR spectra were recorded with a Varian INOVA (500 MHz for ¹H-NMR) spectrometer. Measurements were carried out in deutero-pyridine or deutero-dichloromethane at 30°C. Chemical shifts (δ) are in given in ppm using tetramethylsilane (TMS) as internal standard (δ_{TMS} = 0,00 ppm). The following abbreviations are used in the NMR spectra: s=singlet, d=duplet, t=triplet, q=quartet, m=multiplet, dd=double duplet, br=broad multiplet, dm=duplet multiplet.

High-resolution MS spectra of the compounds were measured by FAB-MS method. Apparatus: Finnigan MAT 95SQ. FAB ionization method: 20 kV Cs⁺ ion gun, 3-nitro-benzylalcohol matrix solvent. Evaluation of spectra: m/z. FAB-daughter-ion spectra were obtained with tandem MS method.

IR spectra were recorded with Perkin-Elmer 1000 spectrophotometer, using potassium bromide pellets, 4 cm⁻¹ spectral resolution.

The following figures are shown to assist the understanding of our invention:
Figure 1: the elution profile of the final product of formula (II) obtained by HPLC. Abscissa: elution time in min, ordinate: absorbency in mAU.
Figure 2: ¹H-NMR spectrum of compound of formula (II) in deutero-pyridine, chemical shifts are given in ppm.
Figure 3: FAB-MS spectrum of compound of formula (II). Abscissa: m/z, ordinate: ion intensity in %.
Figure 4: IR spectrum of compound of formula (II). Abscissa: wavelength (cm⁻¹), ordinate: transmission in %.
Figure 5: the elution profile of the final product of formula (IV) obtained by HPLC. Abscissa: elution time in min, ordinate: absorbency in mAU. (Reference)
Figure 6: ¹H-NMR spectrum of compound of formula (V) in deutero-dichloromethane, chemical shifts are given in ppm. (Reference)
Figure 7: ¹³C-NMR spectrum of compound of formula (V) in deutero-dichloromethane, chemical shifts are given in ppm. (Reference)
Figure 8: FAB-MS spectrum of compound of formula (V). Abscissa: m/z, ordinate: ion intensity in %. (Reference)
Figure 9: FAB-daughter ion spectrum of compound of formula (V). Abscissa: m/z, ordinate: ion intensity in %. (Reference)
Figure 10: IR spectrum of compound of formula (V). Abscissa: wavelength (cm⁻¹), ordinate: transmission in %. (Reference)

### Example 1

### N-{3-[3-(dimethylamino)-1-oxo-2-propenyl]phenyl}-N-ethyl-acetamide hydrochloride of formula (II)

Under nitrogen, 31.5 g of N-{3-[3-(dimethylamino)-1-oxo-2-propenyl]phenyl}-N-ethyl-acetamide of formula (I) was suspended in 300 ml of acetone at room temperature and 10.4 ml of concentrated hydrochloric acid was added dropwise. After adding 0.5 ml of concentrated hydrochloric acid a homogenous solution was obtained, the separation of crystals started after adding 3-4 ml of concentrated hydrochloric acid. After addition of the whole amount of the concentrated hydrochloric acid the reaction mixture was stirred at room temperature for 30 min, and 0-5 °C for further 1 h. The precipitated crystals were filtered off, washed twice with 10 ml of cold (3-5 °C) acetone and dried below 50 °C to yield 31.15 g (87 %) of the title compound.

Melting point: 136-137 °C.

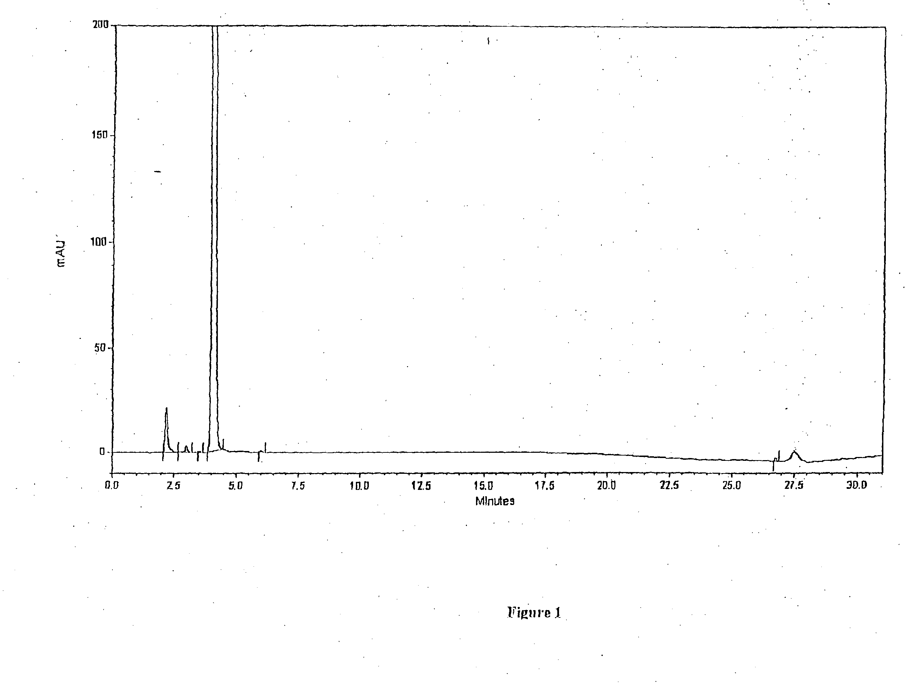

Analytical characterization of the so obtained product:
The elution profile of N-{3-[3-(dimethylamino)-1-oxo-2-propenyl]phenyl}-N-ethyl-acetamide hydrochloride of formula (II) obtained by HPLC is shown in Figure 1. Abscissa: elution time in min, from 0 to 31 min; ordinate: absorbency in mAU. The concentration of the sample was 0.5 mg/ml. The retention time of N-{3-[3-(dimethylamino)-1-oxo-2-propenyl]phenyl}-N-ethyl-acetamide hydrochloride of formula (II) is 4.0 min. The evaluation was carried out by area-normalization.

According to HPLC the purity of the product was 98.5 %.

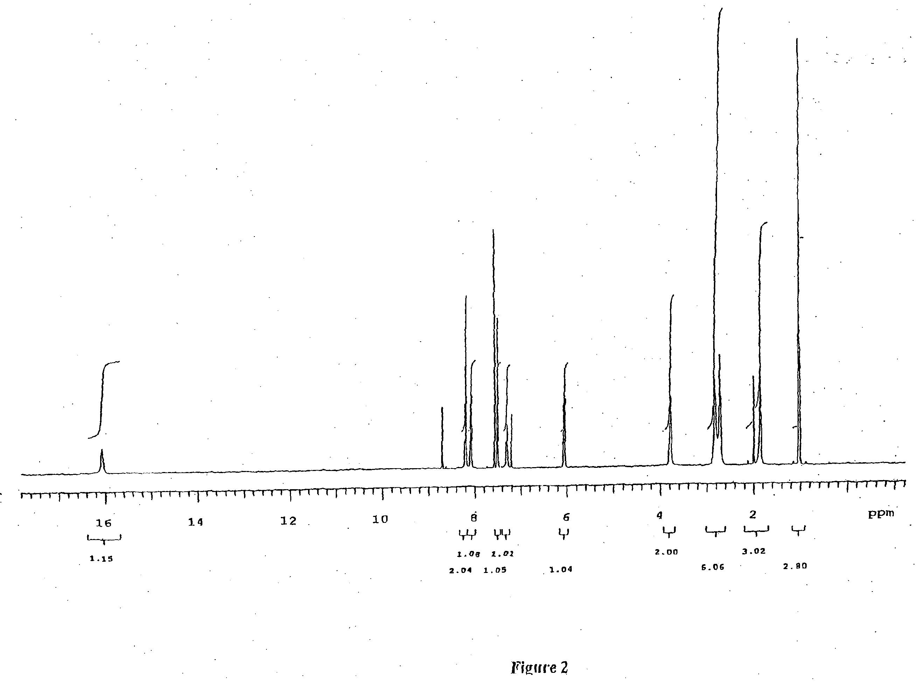

The ¹H-NMR spectrum of compound of formula (II) in deutero-pyridine is shown in Figure 2, where the chemical shifts (δ) are in given in ppm using tetramethylsilane (TMS) as internal standard (δ_{TMS} = 0,00 ppm).

Characteristic chemical shifts are the following:
*¹H-NMR:* 1.03 (t, 3H), 1.87 (s, 3H), 2.74 (s,br, 3H), 2.84 (s,br, 3H), 3.79 (q,br, 2H), 6.07 (d, ³J_{H,H}=12.5Hz, 1H), 7.29-7.33 (m,br, 1H), 7.50-7.54 (m, 1H), 8.10 (d, ³J_{H,H}=12.5Hz, 1H) 8.19-8.23 (m, 2H); 16.07 (s,br, 1H).

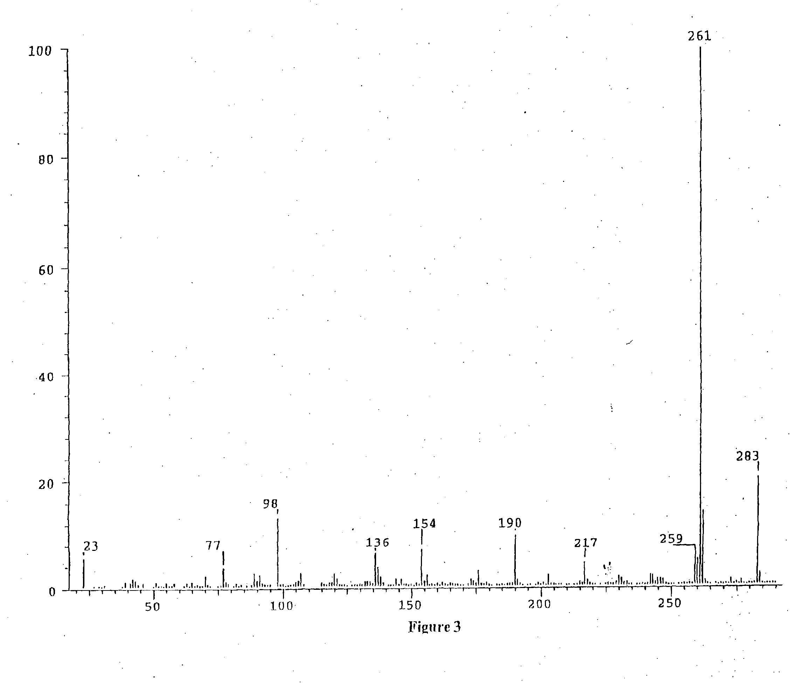

FAB-MS spectrum of compound of formula (II) is shown in Figure 3. The characteristic protonated molecule ion is m/z =261, while the molecule ion with sodium cation is m/z = 283. Other fragment peaks: formation of 3-(dimethylamino)-1-oxo-2-propenyl ion (m/z = 98); cleavage of 2-dimethylamino-ethylene side-chain (m/z = 190), cleavage of acetyl group (m/z = 217), elimination of ethane from the protonated molecule ion (m/z = 231). M/z = 77, 136 and 154 peaks can be derived from the matrix solvent.

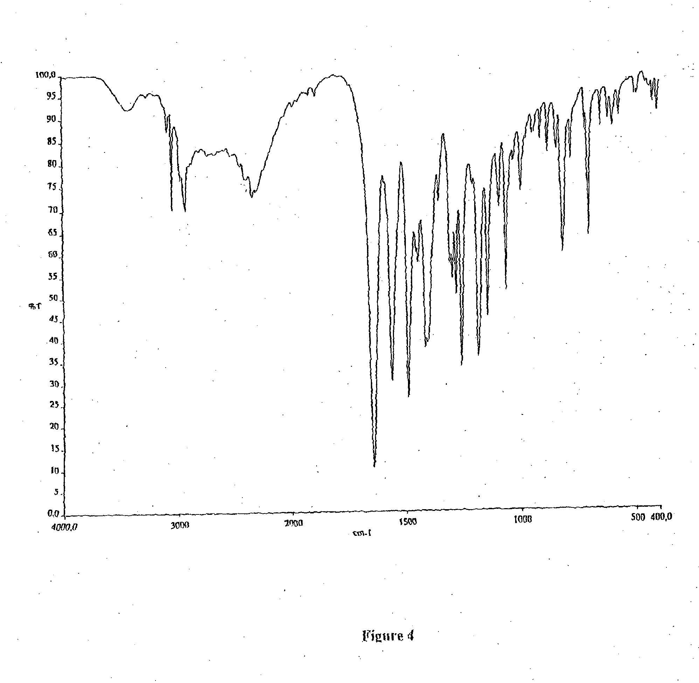

The IR spectrum of compound of formula (II) is shown in Figure 4. The characteristic IR absorption bands (cm⁻¹) are the following:
- N⁺ - H: 2700-2000
- C=O: 1645
- Ar: 820, 706

Further characteristic absorption bands (cm⁻¹): 3050, 2932, 1562, 1492, 1259, 1185, 1146, 1067.

### Example 2 (Reference)

### N-[3-(3-cyanopyrazolo[1,5-a]pyrimidin-7-yl)-phenyl]-N-ethyl-acetamide of formula (IV)

Nitrogen was bubbled through a mixture of 150 ml of purified water and 50 ml of ethanol and under nitrogen 10.8 g 3-amino-4-pyrazole-carbonitrile of formula (III) was added to this mixture. After dissolution the reaction mixture was warmed to 45-50 °C and 29.6 g of N-{3-[3-(dimethylamino)-1-oxo-2-propenyl]phenyl}-N-ethyl-acetamide hydrochloride of formula (II) was added in three equal portions in every 15 min. The reaction mixture, which was a solution in the beginning and later became a suspension, was stirred at this temperature for 1 h under nitrogen. Then the so obtained suspension was cooled to 0-5 °C and stirred at this temperature for 2 h. The precipitated crystals were filtered off, washed with 25 ml of ice-cold ethanol and 2x25 ml of ice-cold water. The product was dried in vacuum below 50 °C. The so obtained product was dissolved in 280 ml of methanol under nitrogen at 65-67 °C in order to remove mechanical impurities. The solution was filtered and the filtrate was cooled to 0-5 °C. The mixture was kept at this temperature for 2 h, then the precipitated crystals were filtered off, washed with 2x10 ml of ice-cold methanol and dried below 50 °C to yield 28.2. g (92.5 %) of the title compound.

Analytical characterization of the so obtained product:

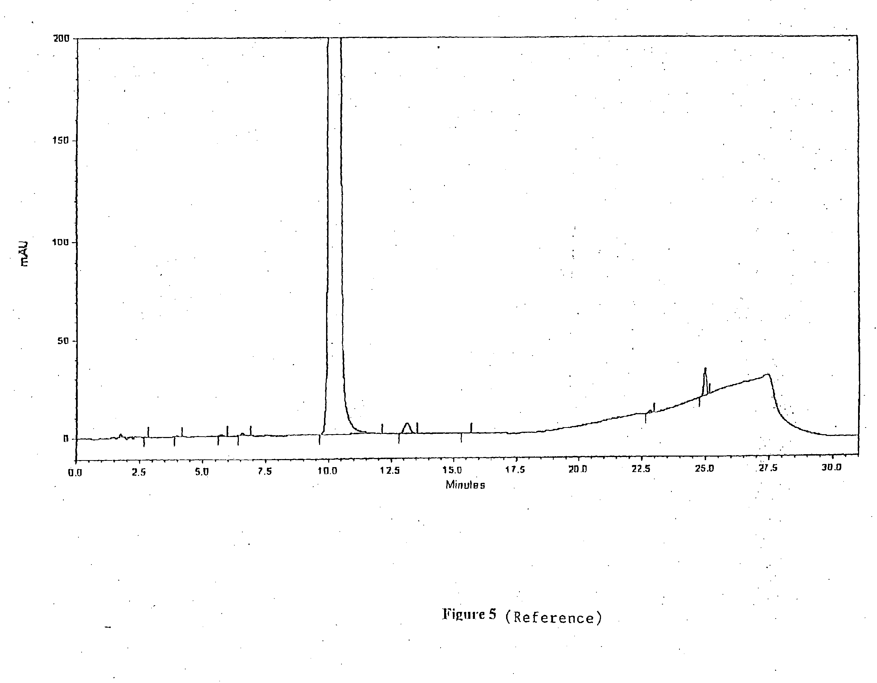

The elution profile of the compound of formula (IV) obtained by HPLC is shown in Figure 5. Abscissa: elution time in min, from 0 to 31 min; ordinate: absorbency in mAU. The concentration of the sample was 1.0 mg/ml. The retention time of the compound of formula (IV) is 10.2 min. The evaluation was carried out by external standard method, thousand-fold dilution of the standard sample solution.

According to HPLC the purity of the product of formula (IV) was 99.8 %.

The retention time of N-{3-[3-cyanopyrazolo(1,5-a)pyrimidin]-6-yl-3-[(3-N-ethyl-acetamido-phenyl)-3-oxo-propene-1-yl] - (pyrimidin-7-yl) -phenyl} -N-ethyl-acetamide of formula (V), which is an impurity formed in 0.07 % yield and was characterized by us, is 24.9 min, as it can be seen in Figure 5.

This impurity was also isolated from the crude final product obtained according to the known procedures for the synthesis of compound of formula (IV), the final products of these procedures contained larger amount (0.1 %) of the impurity of formula (V).

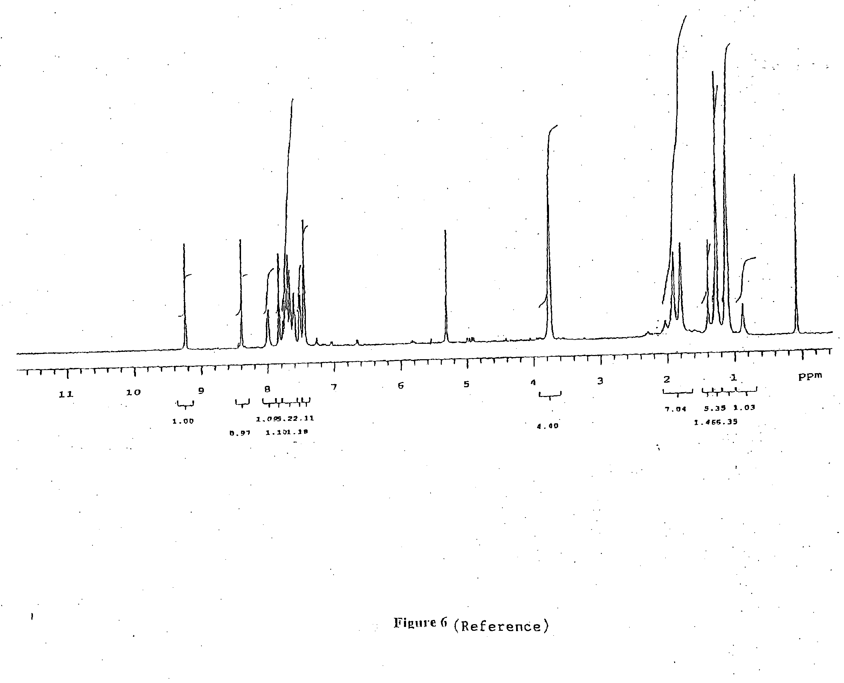

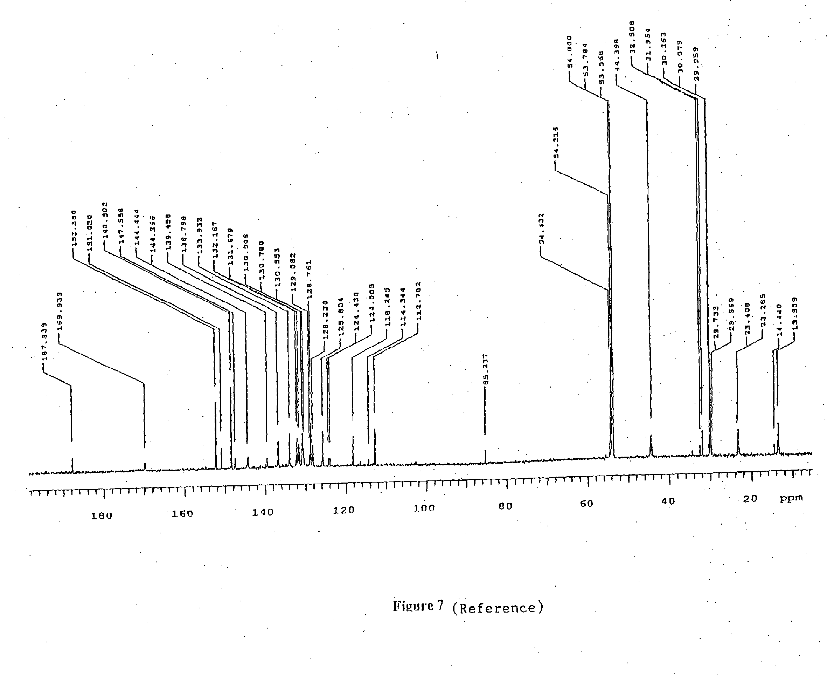

The ¹H-NMR and ¹³C-NMR spectra of compound of formula (V) in deutero-dichloromethane are shown in Figure 6 and Figure 7, where the chemical shifts (δ) are in given in ppm using tetramethylsilane (TMS) as internal standard (δ_{TMS} = 0,00 ppm).

Characteristic chemical shifts of compound of formula (V) are the following:
*¹H-NMR:* 1.12 (t,br, 3H), 1.27 (t,br, 3H), 1.79 (s,br, 3H), 3.76 (q,br, 4H), 7.43-7.86 (m, 9H), 7.98-8.02 (m,br, 1H), 8.40 (s, 1H), 9.24 (s, 1H).
*¹³C-NMR:* 13.5 (CH₃); 22.3 (CH₃), 23.4 (CH₃); 44.4 (CH₂), 44.5 (CH₂); 85.2 (C); 112.8 (C); 118.2 (C); 125.8 (CH); 128.2 (CH); 128.8 (CH); 129.1 (C); 130.6 (CH); 130.8 (CH); 130.9 (CH); 131.7 (CH); 132.2 (CH); 133.9 (CH); 136.8 (CH); 139.4 (C); 144.3 (C); 144.4 (C); 147.6 (C); 148.5 (CH); 151.0 (C); 152.4 (CH); 169.8 (C), 169.9 (C), 187.8 (C).

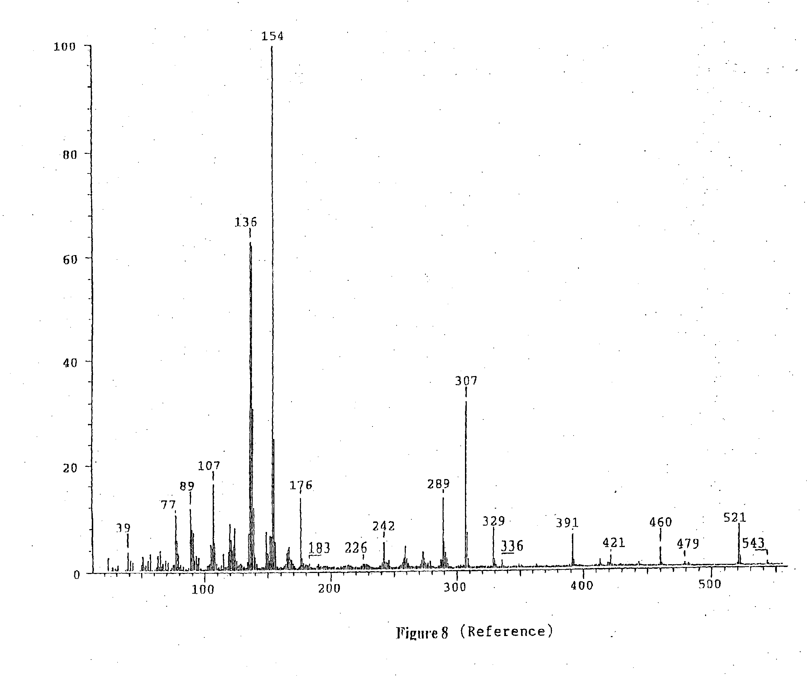

The FAB-MS spectrum of compound of formula (V) is shown in Figure 8. The characteristic protonated molecule ion is m/z =521. M/z = 107, 136, 137, 154, 289 and 307 peaks can be derived from the matrix solvent.

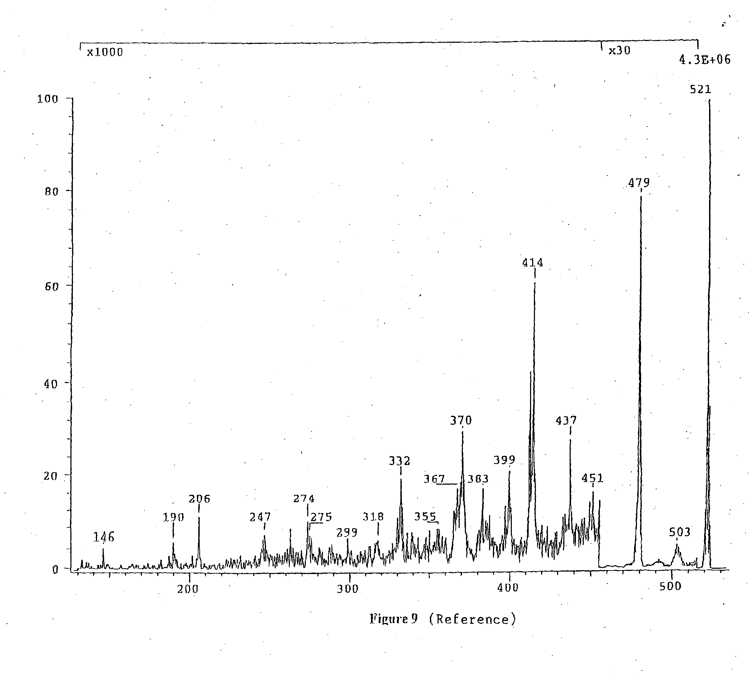

The FAB-daughter ion spectrum of compound of formula (V) is shown in Figure 9. The fragment peaks (m/z = 479 and 437) can be derived from the protonated molecule ion by elimination of one or two ketene groups, respectively.

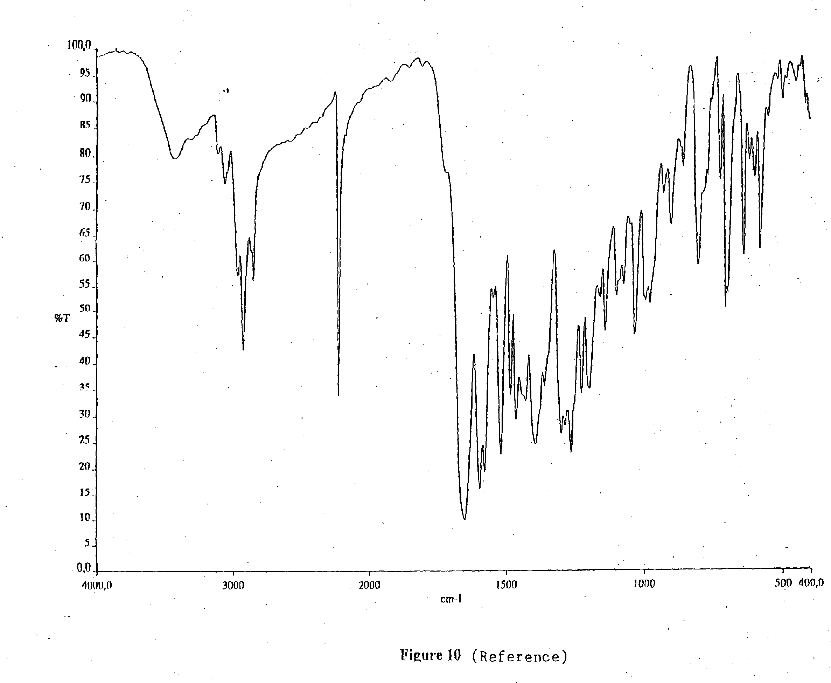

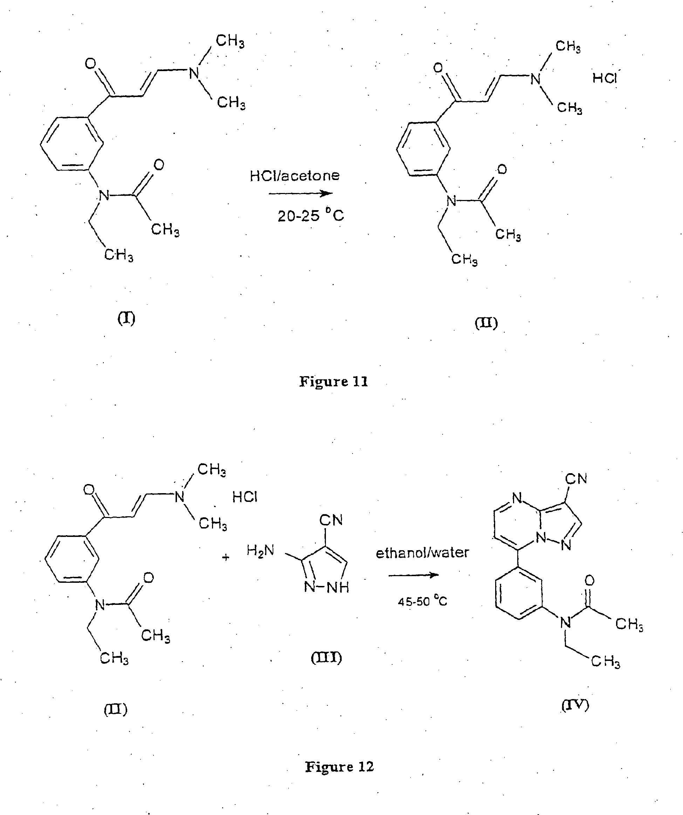

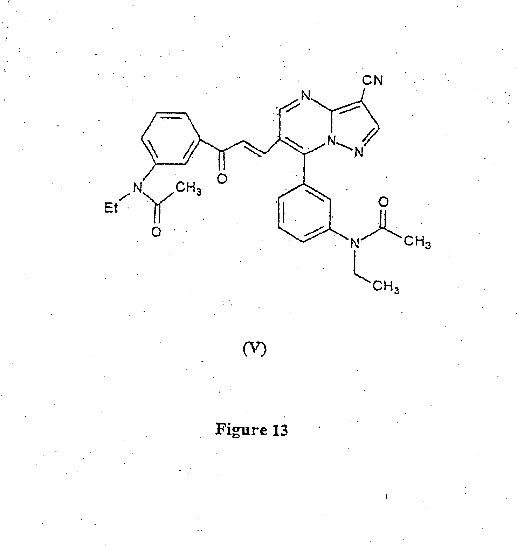

The IR spectrum of compound of formula (V) is shown in Figure 10. The characteristic IR absorption bands (cm⁻¹) are the following:

| | |
|---|---|
| C=N | 2230 |
| C = O | 1652 |
| Ar | 1597, 1579, 807, 707 |

Further characteristic absorption bands (cm⁻¹): 2926, 2854, 1520, 1394, 1265, 1199, 1035, 641, 583.

## Claims

1. N-{3-[3-(dimethylamino)-1-oxo-2-propenyl]phenyl}-N-ethyl-acetamide hydrochloride of formula (II) having a purity of more than 99.5 %.

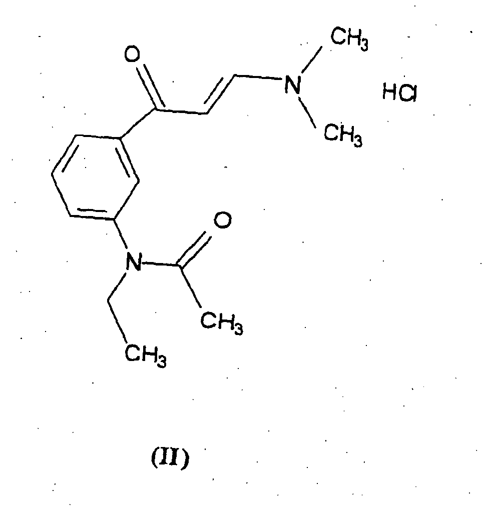

2. Process for the synthesis of N-{3-[3-(dimethylamino)-1-oxo-2-propenyl]phenyl}-N-ethyl-acetamide hydrochloride of formula (II) having a purity of more than 99.5 %, **characterized by** suspending N-{3-[3-(dimethylamino)-1-oxo-2-propenyl]phenyl}-N-ethyl-acetamide of formula (I) in acetone under nitrogen at 20-25°C and adding equimolar amount of concentrated hydrochloric acid to the suspension.

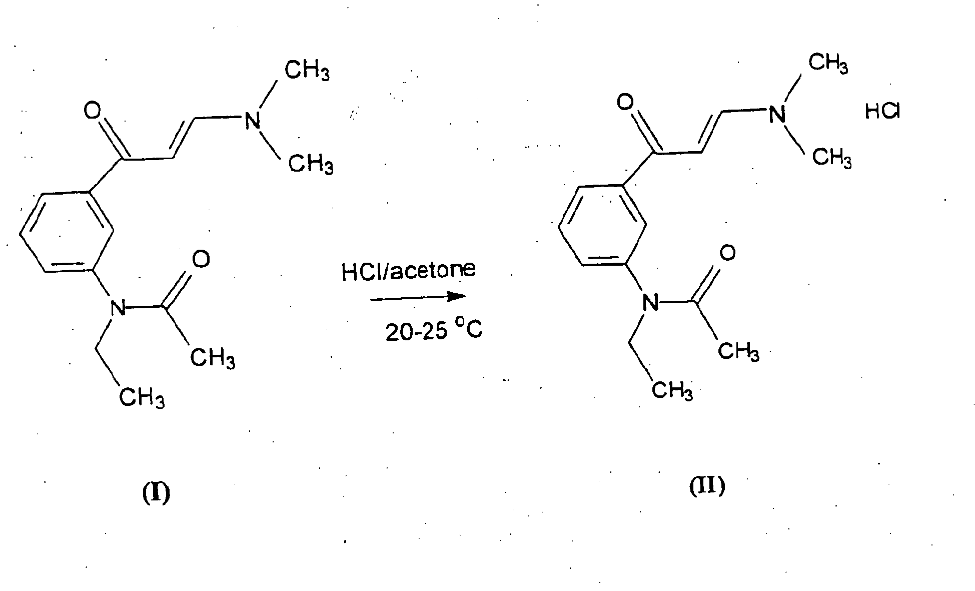

## Patentansprüche

1. N-{3-[3-(Dimethylamino)-1-oxo-2-propenyl]phenyl)-N-ethylacetamid-Hydrochlorid der Formel (II) mit einer Reinheit von mehr als 99,5 %.

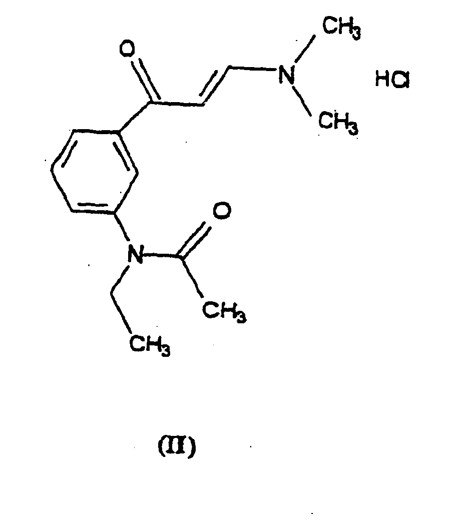

2. Verfahren zur Synthese von N-{3-[3-(Dimethylamino)-1-oxo-2-propenyl]phenyl}-N-ethylacetamid-Hydrochlorid der Formel (II) mit einer Reinheit von mehr als 99,5 %, **gekennzeichnet durch** Suspendieren von N-{3-[3-(Dimethylamino)-1-oxo-2-propenyl]phenyl}-N-ethylacetamid der Formel (I) in Aceton unter Stickstoff bei 20-25°C und Zugeben einer äquimolaren Menge konzentrierter Salzsäure zu der Suspension.

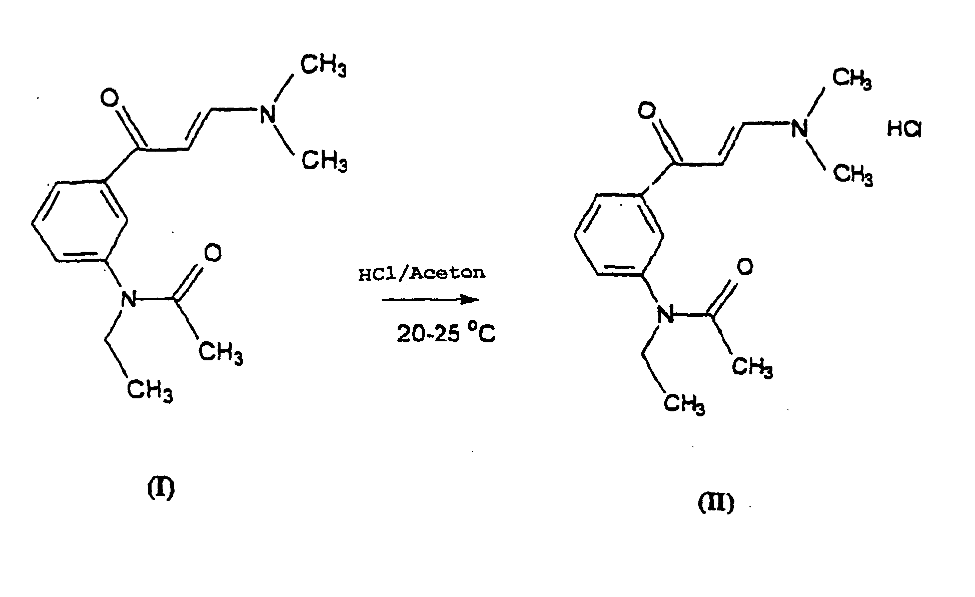

## Revendications

1. Chlorhydrate de N-{3-[3-(diméthylamino)-1-oxo-2-propényl]phényl}-N-éthyl-acétamide de formule (II) ayant une pureté supérieure à 99,5 %.

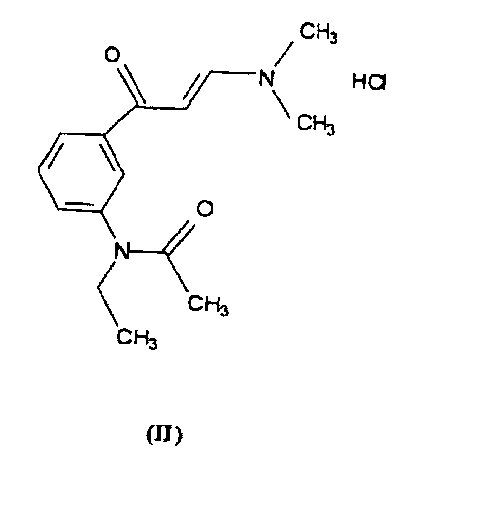

2. Procédé de synthèse de chlorhydrate de N-{3-[3-(diméthylamino)-1-oxo-2-propényl]phényl}-N-éthyl-acétamide de formule (II) ayant une pureté supérieure à 99,5 %, **caractérisé par** les étapes consistant à mettre en suspension du N-{3-[3-(diméthylamino)-1-oxo-2-propényl]phényl}-N-éthyl-acétamide de formule (I) dans de l'acétone sous azote à 20 à 25 °C et ajouter une quantité équimolaire d'acide chlorhydrique concentré à la suspension.
